**Europäisches Patentamt**

**European Patent Office**

**Office européen des brevets**

(11) Veröffentlichungsnummer: **0 275 553 B1**

(12) **EUROPÄISCHE PATENTSCHRIFT**

(45) Veröffentlichungstag der Patentschrift:
**10.10.90**

(21) Anmeldenummer: **87119300.9**

(22) Anmeldetag: **29.12.87**

(51) Int. Cl.⁵: **A61F 2/60**

(54) **Künstlicher gelenkloser Fuss.**

(30) Priorität: **29.12.86  DE 3644613**

(43) Veröffentlichungstag der Anmeldung:
**27.07.88 Patentblatt 88/30**

(45) Bekanntmachung des Hinweises auf die Patenterteilung:
**10.10.90 Patentblatt 90/41**

(84) Benannte Vertragsstaaten:
**AT BE CH DE ES FR GB GR IT LI LU NL SE**

(56) Entgegenhaltungen:
**EP-A- 0 122 372**
**US-A- 3 098 239**

**Die Akte enthält technische Angaben, die nach dem Eingang der Anmeldung eingereicht wurden und die nicht in dieser Patentschrift enthalten sind.**

(73) Patentinhaber: **Otto Bock Orthopädische Industrie Besitz- und Verwaltungs-Kommanditgesellschaft, Industriestrasse, D-3408 Duderstadt 1(DE)**

(72) Erfinder: **Näder, Max, Dr.-Ing. E.h., Hindenburgring 39, D-3408 Duderstadt(DE)**

(74) Vertreter: **Lins, Edgar, Dipl.-Phys. et al, Patentanwälte Gramm + Lins Theodor-Heuss-Strasse 2, D-3300 Braunschweig(DE)**

ACTORUM AG

**Beschreibung**

Die Erfindung betrifft einen künstlichen gelenklosen Fuß mit einem inkompressiblen Kern, vorzugsweise aus Holz, dessen Unterseite von vorn nach hinten schräg ansteigend verläuft und der im Knöchelbereich eine obere Anschlußfläche bildet und sich mit einem nach vorn in der Höhe abnehmenden Teil bis in den Spannbereich des Fußes erstreckt, einem an die Unterseite des Kerns angeschlossenen Fersenkeil aus einem weichen Kunstoffschaum und einer die Anordnung aus Kern und Kunststoffschaum mit Ausnahme der oberen Anschlußfläche vollständig umgebenden hautbildenden Schicht aus Kunststoffschaum.

Ein derartiger künstlicher gelenkloser Fuß ist durch die DE-PS 33 09 777 bekannt. Er weist den wesentlichen Vorteil auf, daß die hautbildende Schicht den Innenaufbau vor Eindringen von Feuchtigkeit und aggressiven Materialien schützt. Der inkompressible Kern erstreckt sich sehr weit in den Spannbereich hinein und endet etwa im vorderen Spannbereich des künstlichen Fußes. An seiner Vorderseite schließt sich lediglich die hautbildende Schicht an, die die Flexibilität des Fußes im Zehen- und vorderen Spannbereich zusammen mit einer an der Unterseite der Vorderkante des Kerns befestigten flächigen Gewebeeinlage bestimmt, die sich bis in die Zehen erstreckt. Die Gewebeeinlage dient zur Stabilisierung des Fußes vor Verwindungen im vorderen Spannbereich. Der im Fersenbereich unter dem Kern angesetzte weiche Fersenkeil wird ebenfalls von der hautbildenden Schicht umschlossen. Diese darf aufgrund ihrer relativ großen Härte (Raumgewicht des Kunststoffschaums ca. 6 g/cm³) im Bereich des Fersenkeils nur eine geringe Wandstärke von ca. 2 mm aufweisen, um die elastischen Eigenschaften des sehr weichen Fersenkeils nicht zu stark zu beeinträchtigen. Der Abrollvorgang mit diesem Fuß wird zunächst durch den weichen, von einer relativ harten Wand umgebenen Fersenkeil und anschließend durch die Biegung der hautbildenden Schicht im vorderen Spannbereich und im Zehenbereich des Fußes bestimmt. Durch den bis in den vorderen Spannbereich erstreckten inkompressiblen Kern ergibt sich eine relative Starrheit des Fußes.

Durch einen Produktkatalog der Campbell Childs, Inc., Phoenix, Oregon, USA, ist ein als S.A.F.E. Foot bezeichneter Fußaufbau bekannt, bei dem ein inkompressibler Kern nur im Knöchelbereich vorgesehen ist. An die Vorderseite dieses Kerns schließt sich ein langgestreckter Innenfuß an, der sich bis in den Zehenbereich erstreckt. Etwa im Bereich der Fußmitte ist der Innenfuß an seiner Unterseite ausgehöhlt, so daß an dieser Stelle eine merkliche Materialverdünnung existiert, die einen Biegebereich für den Innenfuß festlegt. Der Innenfuß und der Kern sind - mit Ausnahme der oberen Anschlußfläche - von einem relativ steifen Schaum umgeben, der zugleich den Fersenkeil bildet. Elastische Bandstreifen erstrecken sich vom Kern unter den Innenfuß und sind an der Spitze des Innenfußes mit diesem verankert. Ein weiterer elastischer Bandstreifen erstreckt sich von der Vorderseite des Kerns direkt in das Material des Innen fußes hinein. Dieser Fuß bietet gegenüber dem eingangs erwähnten Fuß den Vorteil einer Flexibilität auch im Spannbereich, ist jedoch aufgrund seiner Stabilisierungsmaßnahmen durch die Bandstreifen außerordentlich aufwendig. Darüber hinaus ist der Innenaufbau des Fußes nicht gegen das Eindringen von Wasser und aggressiven Agenzien gesichert.

Der Erfindung liegt demgegenüber die Aufgabe zugrunde, einen künstlichen gelenklosen Fuß der eingangs erwähnten Artso auszubilden, daß er mit einem einfachen Aufbau ohne Verstärkungsbänder o. ä. einen einwandfreien Abrollvorgang gewährleistet und dabei eine Flexibilität auch im Spannbereich aufweist.

Diese Aufgabe wird erfindungsgemäß dadurch gelöst, daß sich der Kern von seiner Vorderkante auf der Anschlußfläche aus um weniger als die Hälfte seiner Länge auf der Anschlußfläche in den Spannbereich erstreckt, daß sich an die Vorderfläche des Kerns und an die Vorderseite des Fersenkeils ein sich bis in den Zehenbereich erstreckender flexibler Innenfuß aus Kunststoffschaum anschließt, der ebenfalls von der hautbildenden Schicht eingeschlossen ist und daß die hautbildende Schicht eine weiche, leicht verformbare Kunststoffschaumschicht ist, deren Rückstellkräfte klein gegenüber den Rückstellkräften des Innenfußes sind.

Der erfindungsgemäße Fuß weist einen bisher schon bekannten weichen Fersenkeil auf. Die diesen Fersenteil sowie den erfindungsgemäß vorgesehenen Innenfuß umgebende hautbildende Schicht ist sehr weich ausgebildet und beeinträchtigt die elastischen Eigenschaften des Fersenkeils sowie des Innenfußes praktisch nicht. Demgemäß werden die Laufeigenschaften in der zweiten Phase der Abrollbewegung (beim Anheben der Ferse) praktisch ausschließlich von den flexiblen Eigenschaften des Innenfußes bestimmt. Die hautbildende Schicht hat nur noch die Aufgabe des Schutzes vor eindringendem Wasser und vor eindringenden aggressiven Agenzien sowie der kosmetischen Ausbildung der Fußoberfläche. Im Gegensatz zu der bisher bekannten hautbildenden Schicht werden die Laufeigenschaften des Fußes praktisch nicht mehr durch die hautbildende Schicht beeinflußt.

Der Innenfuß des erfindungsgemäßen künstlichen Fußes ist kürzer ausgebildet als der bekannte Innenfuß der Firma Campbell Childs. Er ist - mit Ausnahme des Zehenbereichs - von der als flächige Umhüllungsschicht ausgebildeten hautbildenden Schicht umgeben, weist also keine Unregelmäßigkeiten in seiner Kontur auf. Seine Form entspricht im wesentlichen der Form des Vorderfußes im Spann- und Knöchelbereich.

Der erfindungsgemäße Fuß hat den wesentlichen Vorteil, völlig ohne zusätzliche Versteifungsmittel, wie elastische Metallbänder o. ä., auszukommen. Daher gestaltet sich sein Aufbau einfach und seine Herstellung preiswert. Er weist bereits im Spannbereich eine ausreichende Flexibilität auf und gestattet daher die Anpassung an eigere Schuhe usw.

In einer erprobten Ausführungsform weist der Kunstoffschaum der hautbildenden Schicht ein Raumgewicht von etwa 4 g/cm³, der Innenfuß ein Raumgewicht von ca. 6 -10 g/cm³ und der Fersenkeil ein Raumgewicht von ca.2-4 g/cm³ auf. Dabei ist vorzugsweise der Kern sehr kurz ausgebildet, so daß er sich von seiner Vorderkante auf der Anschlußfläche aus um weniger als ein Drittel seiner Länge auf der Anschlußfläche in den Spannbereich erstreckt.

Vorzugsweise erstreckt sich bei dem erfindungsgemäßen Fußaufbau der Fersenkeil mit seiner vorderen Spitze etwa bis zur vorderen Kante des Kerns, also bis in das Fußgewölbe hinein. Dadurch wird eine Federung über einen sehr weiten Bereich des Abrollvorgangs erzielt.

Die Erfindung soll im folgenden anhand eines in der Zeichnung dargestellten Ausführungsbeispiels näher erläutert werden. Es zeigen:

Figur 1 - einen Längsschnitt durch einen künstlichen Fuß entlang der Linie I-I in Figur 2

Figur 2 - eine Draufsicht auf den Fuß gemäß Figur 1

Figur 3 - einen Querschnitt durch den Fuß gemäß Figur 1 entlang der Linie III in Figur 1.

Der in der Zeichnung dargestellte künstliche Fuß besteht im wesentlichen aus einem Kern 1 aus Holz, einem Fersenkeil 2, einem Innenfuß 3 sowie einer hautbildenden Schicht 4, die den gesamten Fuß - mit Ausnahme einer oberen Anschlußfläche 5 des Kerns 1 - umschließt.

Der Kern 1 ist in an sich bekannter Weise mit einer von vorn nach hinten schräg ansteigenden Unterseite 6 versehen, die den Plazt für den im Längsschitt im wesentlichen dreieckigen Fersenkeil 2 schafft. Der Fersenkeil 2 besteht aus einem weichen Kunstoff-Schaum mit einem Raumgewicht von ca. 3 g/cm³.

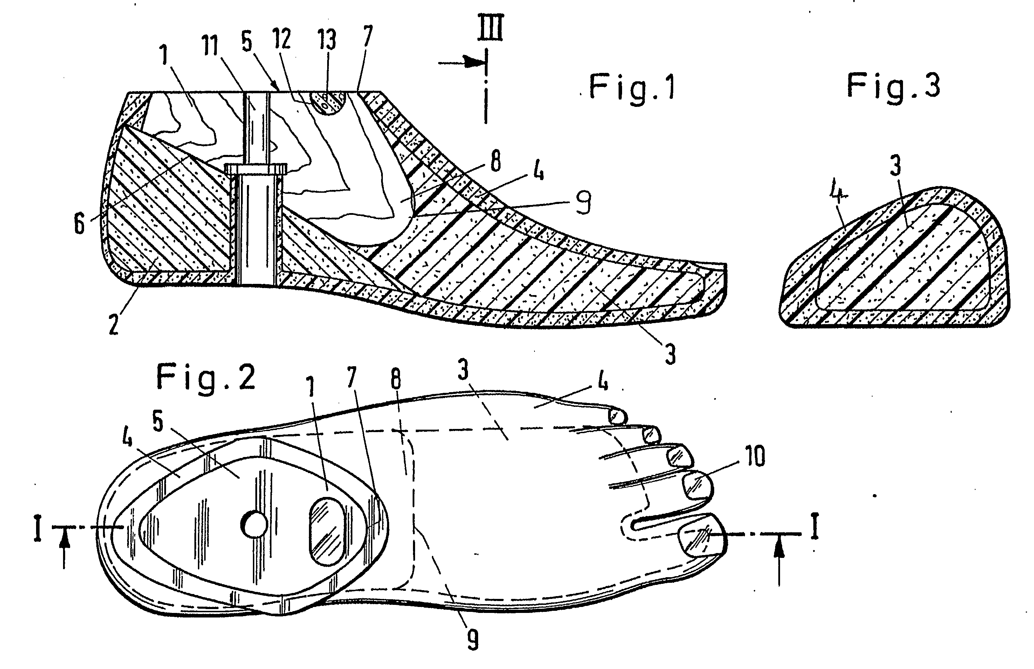

Der Kern 1 ragt von seiner Vorderkante 7 der Anschlußfläche 5 aus mit einem Schräg nach unten zeigenden, sich abgerundet verjüngenden Ansatz 8 in den Spann-Bereich des Fußes. Der Abstand von der vorderen Kante 9 des Ansatzes 8 zur Vorderkante 7 der Anschlußfläche 5 beträgt in dem dargestellten Ausführungsbeispiel etwa ein Fünftel der maximalen Länge des Kerns 1 auf der Anschlußfläche 5. Der sich an die Kontur des Ansatzes 8 anschließende Innenfuß 3 erstreckt sich gleichförmig bis in den Zehenbereich des Fußes. Seine Form entspricht etwa der Form des Fußes, da die hautbildende Schicht 4 den Innenfuß allseitig als flächige Umgebungs schicht umgibt, wobei Wandstärkenvariationen, wie sie beispielsweise in Figur 3 erkennbar sind, im wesentlichen aus kosmetischen Gründen vorkommen. Die Schicht 4 umfaßt den gesamten Fuß praktisch als flächige Umgebungsschicht, wenn man von der Ausbildung der Zehen 10 absieht.

Kern 1 und Fersenkeil 2 durchzieht eine Bohrung 11, die sich am unteren Rand des Kerns 1 stufenförmig erweitert und zur Aufnahme einer Befestigungsschraube mit Schraubenkopf zur Anbringung an ein künstliches Bein dient. Im Bereich des größeren Durchmessers ist die Bohrung 11 ebenfalls mit einer dünnen Wand der Hautbildenden Schicht 4 versehen, so daß auch von der Bohrung 11 her in den Innenaufbau des Fußes keine Feuchtigkeit oder aggressive Agenzien eindringen können. Die Abdichtung zum Holzkern 1 erfolgt durch eine einlegbare Dichtscheibe aus Kunststoff.

Auf der Anschlußfläche 5 weist der Kern eine Aushöhlung 12 auf, die mit einem gefüllten Gießharz 13 ausgefüllt ist. Die Ausnehmung 12 befindet sich an der Stelle, die von einem Anschlußteil beim Abrollen des Fußes regelmäßig beaufschlagt wird, so daß an dieser Stelle normalerweise ein erhöhter Verschleiß des Kerns 1 zu beobachten ist. Diesem Verschleiß wird durch die Einbringung des gefüllten Gießharzes entgegengewirkt.

**Patentansprüche**

1. Künstlicher gelenkloser Fuß mit einem inkompressiblen Kern (1), vorzugsweise aus Holz, dessen Unterseite (6) von vorn nach hinten schräg ansteigend verläuft und der im Knöchelbereich eine obere Anschlußfläche (5) bildet und sich mit einem nach vorn in der Höhe abnehmenden Ansatz (8) bis in den Spannbereich des Fußes erstreckt, einem an die Unterseite (6) des Kerns angeschlossenen Fersenkeil (2) aus einem weichen Kunststoffschaum und einer die Anordnung aus Kern (1) und Fersenkeil (2) mit Ausnahme einer oberen Anschlußfläche (5) vollständig umgebenden hautbildenden Schicht (4) aus einem Kunststoffschaum, dadurch gekennzeichnet, daß sich der Kern (1) von seiner Vorderkante (7) auf der Anschlußfläche (5) aus um weniger als die Hälfte seiner Länge auf der Anschlußfläche (5) in den Spann bereich erstreckt, daß sich an die Vorderfläche des Kerns (1) und an die Vorderseite des Fersenkeils (2) ein sich bis in den Zehenbereich erstreckender flexibler Innenfuß (3) aus Kunststoffschaum anschließt, der ebenfalls von der hautbildenden Schicht (4) eingeschlossen ist und daß die hautbildende Schicht (4) eine weiche, leicht verformbare Kunststoffschaumschicht ist, deren Rückstellkräfte klein gegenüber den Rückstellkräften des Innenfußes (3) sind.

2. Künstlicher Fuß nach Anspruch 1, dadurch gekennzeichnet, daß der die hautbildende Schicht (4) bildende Kunststoffschaum ein Raumgewicht von etwa 4 g/cm³ aufweist.

Künstlicher Fuß nach Anspruch 1 oder 2, dadurch gekennzeichnet, daß der Innenfuß (3) ein Raumgewicht von ca. 6 -10 g/cm³ aufweist.

4. Künstlicher Fuß nach einem der Ansprüche 1 bis 3, dadurch gekennzeichnet, daß der Fersenkeil (2) ein Raumgewicht von ca.2-4g/cm³ aufweist.

5. Künstlicher Fuß nach einem der Ansprüche 1 bis 4, dadurch gekennzeichnet, daß sich der Kern (1) von seiner Vorderkante (7) auf der Anschlußfläche (5) aus um weniger als ein Drittel seiner Länge auf der Anschlußfläche (5) in den Spannbereich erstreckt.

6. Künstlicher Fuß nach einem der Ansprüche 1 bis 5, dadurch gekennzeichnet, daß seine elastischen Eigenschaften ausschließlich durch den Innenfuß (3), den Fersenkeil (2) und die Schicht (4) bestimmt sind.

7. Künstlicher Fuß nach einem der Ansprüche 1 bis 6, dadurch gekennzeichnet, daß die Schicht (4) an allen Teilen des Fußes mit Ausnahme der Zehen (10) im wesentlichen als flächige Umhüllungsschicht ausgebildet ist.

8. Künstlicher Fuß nach einem der Ansprüche 1 - 7, dadurch gekennzeichnet, daß der Fersenkeil (2) mit seiner vorderen Spitze etwa mit der vorderen Kante des Kerns (1) abschließt.

## Claims

1. Artifical jointless foot having an incompressible core (1), preferably of wood, whose underside (6) extends from front to back rising at an incline and which forms an upper connecting surface (5) in the region of the ankle and extends with a shoulder (8) decreasing forwardly in height into the region of the instep of the foot, the heel wedge (2) consisting of soft plastics foam connected to the underside (6) of the core, and a skin-forming layer (4) consisting of plastics foam which completely surrounds the arrangement composed of the core (1) and heel wedge (2) with the exception of the upper connecting surface (5), characterised in that the core (1) extends from its front edge (7) on the connecting surface (5) into the region of the instep by less than half its length on the connecting surface (5), the front surface of the core (1) and the front face of the heel wedge (2) are adjoined by a flexible inner foot (3) of plastics foam extending into the region of the toes and likewise enclosed by the skin-forming layer (4), and the skin-forming layer (4) is a soft, readily deformable plastics foam layer whose powers of restoration are small compared with the powers of restoration of the inner foot (3).

2. Artificial foot according to claim 1, characterized in that the plastics foam forming the skin-forming layer has a weight per unit volume of about $4g/cm^3$.

3. Artificial foot according to claim 1 or 2, characterised in that the inner foot (3) has a weight per unit volume of about $6 - 10g/cm^3$.

4. Artificial foot according to any one of claims 1 to 3, characterised in that the heel wedge (2) has a weight per unit volume of about $2 - 4g/cm^3$.

5. Artificial foot according to any one of claims 1 to 4, characterized in that the core (1) extends from its front edge (7) on the connecting surface (5) into the region of the instep by less than one third of its length on the connecting surface (5).

6. Artificial foot according to any one of claims 1 to 5, characterised in that its elastic properties are determined exclusively by the inner foot (3), the heel wedge (2) and the layer (4).

7. Artificial foot according to any one of claims 1 to 6, characterised in that the layer (4) is in the form essentially of a laminar enveloping layer in all parts of the foot, with the exception of the toes (10).

8. Artificial foot according to any one of claims 1 to 7, characterised in that the heel wedge (2) has its front tip terminating approximately in line with the front edge of the core (1).

## Revendications

1. Pied artificiel non articulé comprenant un noyau incompressible (1), de préférence en bois, dont la face inférieure (6) est en pente oblique ascendante d'avant en arrière et qui, dans la zone de la cheville, forme une surface supérieure (5) de raccordement et s'étend, par une excroissance (8) diminuant de hauteur vers l'avant, jusque dans la zone de cou-de-pied, un coin (2) formant talon, en une mousse synthétique souple, raccordé à la face inférieure (6) du noyau, et une couche (4) formant peau, en une mousse synthétique, entourant complètement constitué par le noyau (1) et le coin (2) formant talon à l'exception d'une surface supérieure (5) de raccordement, caractérisé en ce que le noyau (1) s'étend dans la zone du coup-de-pied, à partir de son bord antérieur (7) sur la surface de raccordement (5), sur une longueur inférieure à la moitié de sa longueur mesurée sur la surface de raccordement (5), en ce qu'à la face antérieure du noyau (1) et au côté antérieur du coin (2) formant talon se raccorde un pied interne flexible (3) en mousse synthétique, qui s'étend jusque dans la zone des orteils et qui est également enserré par la couche (4) formant peau, et en ce que la couche (4) formant peau est une couche de mousse synthétique souple, facilement déformable, dont les forces de rappel sont peu importantes comparées aux forces de rappel du pied interne (3).

2. Pied artificiel conforme à la revendication 1, caractérisé en ce que la mousse synthétique formant la couche (4) formant peau a une masse spécifique d'environ $4g/cm^3$.

3. Pied artificiel conforme à l'une des revendications 1 ou 2, caractérisé en ce que le pied interne (3) a une masse spécifique d'environ 6 à $10g/cm^3$.

4. Pied artificiel conforme à l'une des revendications 1 à 3, caractérisé en ce que le coin (2) formant talon a une masse spécifique d'environ 2 à $4 g/cm^3$.

5. Pied artificiel conforme à l'une des revendications 1 à 4, caractérisé en ce que le noyau (1) s'étend dans la zone du coup-de-pied, à partir de son bord antérieure (7) sur la surface de raccordement (5), sur une longueur inférieure au tiers de sa longueur mesurée sur la surface de raccordement (5).

6. Pied artificiel conforme à l'une des revendications 1 à 5, caractérisé en ce que ses propriétés d'élasticité sont exclusivement définies par le pied interne (3), le coin (2) formant talon et la couche (4).

7. Pied artificiel conforme à l'une des revendications 1 à 6, caractérisé en ce que la couche (4) qui recouvre toutes les parties du pied à l'exception des orteils (10) est constituée principalement comme une enveloppe mince.

8. Pied artificiel conforme à l'une des revendications 1 à 7, caractérisé en ce que le coin (2) formant talon se termine par sa pointe antérieure à peu près au droit du bord antérieure du noyau (1).

Fig.3

Fig.1

Fig.2